Europäisches Patentamt

European Patent Office

Office européen des brevets

(11) Publication number: **0 291 164**
**A2**

## (12) EUROPEAN PATENT APPLICATION

(21) Application number: **88303203.9**

(22) Date of filing: **11.04.88**

(51) Int. Cl.⁴ **C12N 5/00 , A61K 33/30**

(30) Priority: **13.05.87 US 49176**

(43) Date of publication of application:
**17.11.88 Bulletin 88/46**

(84) Designated Contracting States:
**AT BE CH DE ES FR GB GR IT LI LU NL SE**

(71) Applicant: **SPECTRAL BIOANALYSIS LTD.**
**12 York Gate**
**London NW1 4QS(GB)**

(72) Inventor: **Kubler, Ulrich**
**28 Knoebelstrasse**
**D-8000 Munich 22(DE)**

(74) Representative: **Woodcraft, David Charles et al**
**BROOKES & MARTIN High Holborn House**
**52/54 High Holborn**
**London, WC1V 6SE(GB)**

(54) Growth-suppressing infusion medium, growth-suppressing cell culture medium and processes for the suppression of transformed cell growth.

(57) A growth-suppressing infusion medium comprising a basic infusion medium, and additionally, bivalent zinc in an amount sufficiently high to suppress transformed cell growth and a growth-suppressing cell culture medium comprising a basic cell culture medium, and additionally, bivalent zinc in an amount sufficiently high to suppress transformed cell growth. The present invention also relates to processes for suppressing transformed cell growth, either in vitro or in vivo.

## GROWTH-SUPPRESSING INFUSION MEDIUM, GROWTH-SUPPRESSING CELL CULTURE MEDIUM AND PROCESSES FOR THE SUPPRESSION OF TRANSFORMED CELL GROWTH

The present invention relates to a growth-suppressing infusion medium comprising a basic infusion medium, and additionally, bivalent zinc in an amount sufficiently high to suppress transformed cell growth and a growth-suppressing cell culture medium comprising a basic cell culture medium, and additionally, bivalent zinc in an amount sufficiently high to suppress transformed cell growth. The present invention also relates to processes for suppressing transformed cell growth, either in vitro or in vivo.

Tumor-bearing humans and animals possess low serum levels of bivalent zinc, i.e., from about 0.2 mg/l of $Zn^{++}$ to about 0.7 mg/l of $Zn^{++}$ with an average of 0.2 to 0.6 mg/l of $Zn^{++}$. These low serum levels of bivalent zinc have been found to provide poor growth conditions for transformed cells (see U.S. Patent Application Serial No. 918,654, filed October 14, 1986).

In addition, the above-described low serum levels of bivalent zinc, i.e., below about 0.7 mg/l of $Zn^{++}$, adversely affect some zinc-dependent immune functions, such as the migratory ability of granulocytes and the production of antibodies within the plasma cells, which results in a weakened immune response (see News Notes April 85: Annual Meeting of the American Assoc, for the Advancement of Sciences, Los Angeles, May 26-31, 1985; DePasquale-Jardieu, P. et al, J. Nutrition, 114:1762-1769 (1984) and Alcock, N.W. et al, Nutrition Res., Suppl. (1), pp. 244-250 (1985)).

It has been known that in tumor-bearing humans and animals intraoperative and/or postoperative whole blood-infusions stimulate the process of metastasis of the tumors and thereby disadvantageously affect the long-term survival rate of the tumor-bearing humans and animals (see Foster, R.S. et al., Cancer, 55:1195-1201 (1985)).

Whole blood-infusions, e.g., those used intraoperative and/or post-operative, contain relatively large amounts of bivalent zinc (on the order of about 6.5 to 8.5 mg/l) both in their plasma as well as in their white and red blood cells. These relatively large amounts of bivalent zinc result in an increase in the serum level of bivalent zinc and if the serum level of bivalent zinc is increased beyond a level of about 0.8 mg/l of $Zn^{++}$, transformed cell growth will be promoted (see U.S. Patent Application Serial No. 918,654, filed October 14, 1986). While use of bivalent zinc as described in U.S. Patent Application Serial No. 918,654 is quite advantageous where growth of transformed cells is desirable, this increase is particularly disadvantageous in intraoperative and/or post-operative procedures on tumor-bearing humans and animals since a higher long-term risk of metastasis and growth promotion after tumor-exstirpation exists.

It has been found in the present invention that elevated levels of bivalent zinc, i.e., in excess of that which has been found to promote transformed cell growth, suppress transformed cell growth, in vitro and in vivo.

Thus, one aspect of the present invention provides an infusion medium which allows for the suppression of growth of transformed cells.

A second aspect of the invention provides and infusion medium reducing the long-term risk of metastasis after tumor extirpation.

A third aspect of the invention provides a cell culture medium which allows for the suppression of transformed cell growth.

A fourth aspect of the invention provides processes for suppressing the growth of transformed cells either in vitro or in vivo.

According to the invention there is provided a basic infusion medium or a basic cell culture medium, and additionally, bivalent zinc, wherein the bivalent zinc is present in an amount sufficiently high to suppress transformed cell growth, preferably on the order of about 1.6 mg/l of $Zn^{++}$ or greater.

According to the invention there is also provided process for suppressing the growth of transformed cells in vitro comprising culturing transformed cells in the presence of cell culture medium comprising a basic cell culture medium, and additionally, bivalent zinc in an amount sufficiently high to suppress transformed cell growth, preferably on the order of about 1.6 mg/l of $Zn^{++}$ or greater.

According to the invention there is provided bivalent zinc for use as a medicament, especially for the suppression of tumor growth. The present invention also provides a process for suppressing the growth of transformed cells in vivo comprising increasing the serum level of bivalent zinc in a tumor-bearing organism to an amount sufficiently high to suppress growth, i.e., on the order of about 1.6 mg/l of $Zn^{++}$ or greater.

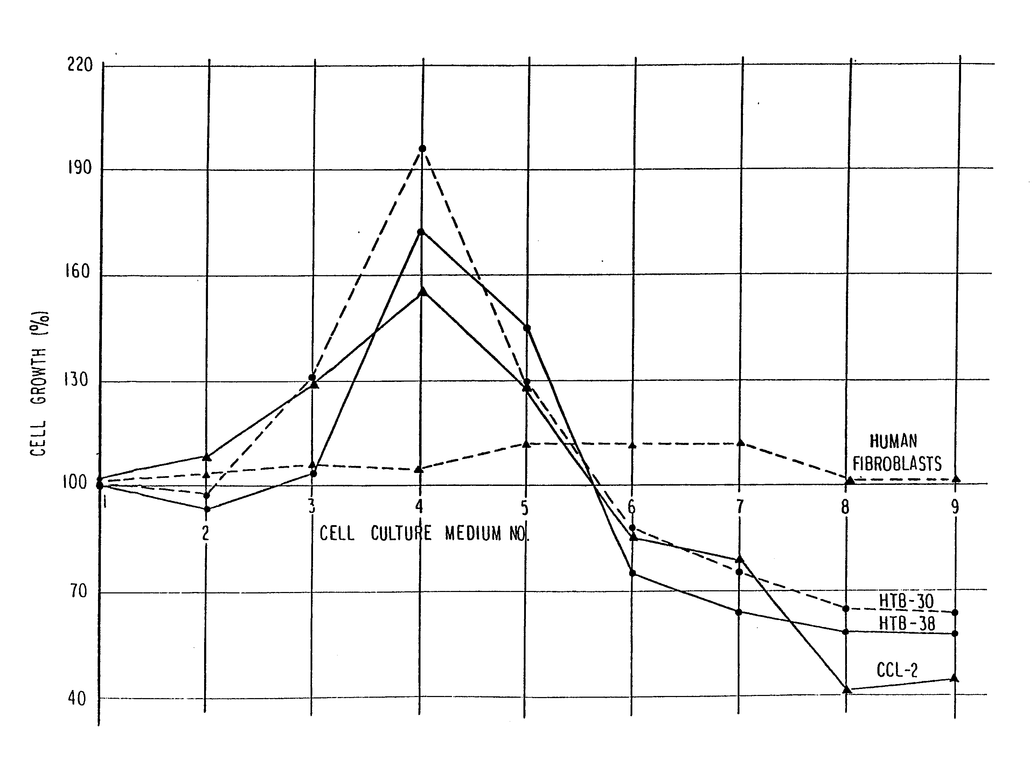

The invention will be illustrated by reference to the accompanying figure which shows the cell growth, expressed by the protein concentration, for the transformed cell lines HTB-38, HTB-30 and CCL-2 and non-transformed human fibroblasts demonstrating cell growth inhibition in accordance with the invention.

As a basic infusion medium, the medium of the present invention can be any conventional infusion medium such as a physiological saline solution, a glucose solution, a fructose solution, whole blood, whole blood fractions, plasma or plasma expanders, preferably a physiological saline solution.

As a basic cell culture medium, the medium of the present invention can be any conventional basic cell culture medium which contains a source of carbon, a source of nitrogen and other inorganic and organic components such as vitamins, amino acids and the like, e.g., as disclosed in H.J. Morton, In Vitro 6:89-108 (1970).

The basic cell culture medium employed in the process of the present invention will depend upon the type of cells being cultured, i.e. mammalian, reptilian, avian or insect, each of which, as is well known to one skilled in the art, has different growth requirements. Further, the basic cell culture medium of the present invention is one which can be employed to culture either transformed cells of mammalian, reptilian, insect or avian origin. Commercially available examples of such basic cell culture mediums, e.g., for mammalian cells, to which the present invention is applicable include HAM F-10, HAM F-12 Eagle's Minimum Essential Medium (MEM), Puck's N-15 and Puck's N-16, McCoy 5A (mod.), RPMI 1603, 16344, 1640, and the like. The preferred basic cell culture medium employed in the present invention is McCoy 5A (mod.).

As defined herein, the term "transformed" cells includes cells of malignant origin (human or non-human).

Examples of such transformed cells include embryonal carcinoma, testis, metastasis to lymph node Cales-1 B (ATCC No. HTB-104), breast HBL-100 (ATCC No. HTB-124), endometrial adenocarcinoma HEC-1-A (ATCC No. HTB-112) Walker rat carcinoma LLC-URC 256 (ATCC No. CCL-38), HT-29 (ATCC No. HTB-38), SK-BR-3 (ATCC No. HTB-30), RT-4 (ATCC No. HTB-2), HT-3 (ATCC No. HTB-32), NCI-H128 (ATCC No. HTB-120), TE-671 (ATCC No. HTB-139), K-562 (ATCC No. CCL-243) and human epithelial cervix carcinoma HeLa (ATCC No. CCL-2).

Basic infusion media which are conventionally employed in the art contain less than 0.01 mg/l of $Zn^{++}$ in the form of $ZnCl_2$.

The infusion medium of the present invention contains bivalent zinc in an amount sufficiently high to suppress transformed cell growth, e.g., preferably about 1.6 mg/l of $Zn^{++}$ or greater, more preferably about 1.6 mg/l of $Zn^{++}$ to about 4.0 mg/l of $Zn^{++}$, and most preferably about 2.1 to 3.5 mg/l of $Zn^{++}$. Levels of $Zn^{++}$ above about 3.5 mg/l do not yield a greater inhibition effect, i.e., a steady state of growth inhibition is reached at levels of $Zn^{++}$ above about 3.5 to 4.0 mg/l.

Basic cell culture media which are conventionally employed in the art contain inorganic and organic components along with trace amounts of bivalent zinc in the form of, e.g., $ZnSO_4$. The amount of the bivalent zinc in such basic culture medium, e.g., HAM F-10 and HAM F-12 (Serva Company) is about 0.007 to 0.2 mg/l of $Zn^{++}$. However, cell culture media containing such trace amounts of bivalent zinc do not give rise to either a growth promoting or growth suppressing effect. It has been found in the present invention that when the amount of bivalent zinc in the culture medium is above trace amounts and above the the above-described minimum, a cell growth suppression effect is achieved.

By employing an amount of bivalent zinc preferably about 1.6 mg/l of $Zn^{++}$ or greater, more preferably 2.1 to 4.0 mg/l of $Zn^{++}$, in conjunction with a conventional cell culture medium, "suppression studies" can be carried out with transformed cells in vitro. As discussed above, levels above 3.5 to 4.0 mg/l of $Zn^{++}$ do not yield a greater inhibition effect, i.e., a steady state of growth inhibition is reached at levels of $Zn^{++}$ above 3.5 to 4.0 mg/l.

These above-described amounts of bivalent zinc are not cytotoxic but can enhance the efficiency of conventional cytostatica in vivo and in vitro so as to establish a test method for measuring the actual need of cytostatica in connection with bivalent zinc in vitro. Since malignant cell growth is decreased by the above-described amounts of bivalent zinc, less amounts of cytostatica are needed, if simultaneously administered with the bivalent zinc, thus increasing the efficiency of the cytostatica.

For the "suppression studies", the transformed cells are cultured in the cell culture medium of the present invention at the optimum known culture conditions for the particular cell, typically at 36.5°C ± 0.5°C, 5% $CO_2$/95% air.

In order to establish a sufficient serum level of bivalent zinc within an organism's blood pool in accordance with the processes of the present invention, the following physiological conditions and mechanisms must be taken into consideration:

(1) In healthy humans and animals, the serum bivalent zinc level is between about 0.8 and 1.5 mg/l of $Zn^{++}$;

(2) In tumor-bearing humans and animals, the serum bivalent zinc level is between about 0.2 and 0.6 mg/l of $Zn^{++}$; and

3

(3) The secretion rate of bivalent zinc is regulated by mineralocorticoid hormones such as aldosteron.

Thus, for therapeutic suppression of malignant cell growth, the amount of bivalent zinc added to a whole blood infusion will depend upon the physiologically available trace amounts of bivalent zinc inherently in the whole blood infusion, so long as a final concentration of about 1.6 to 4.0 mg/l $Zn^{++}$ is reached. To establish the "growth-suppression" bivalent zinc concentrations of the present invention within the blood pool of the body, equivalent amounts of bivalent zinc salts can also be administered using an infusion medium other than whole blood, as long as the final concentration of bivalent zinc achieved in the whole blood pool is within the range of about 1.6 to 4.0 mg/l $Zn^{++}$.

The bivalent zinc used in the present invention can be from any zinc source and the source is not limiting. Suitable sources of the bivalent zinc include any non-toxic inorganic bivalent zinc compound or non-toxic organic bivalent zinc compound. Examples of such non-toxic inorganic bivalent zinc compounds include $ZnCl_2$, $ZnO$, $ZnCO_3$ and $ZnSO_4$. The preferred non-toxic inorganic bivalent zinc compound which can be employed in the present invention is $ZnCl_2$. Examples of such non-toxic organic bivalent zinc compounds which can be employed in the present invention include zinc salts of organic compounds such as zinc acetate. Mixtures of the above-described bivalent zinc compounds can also be employed in the present invention if desired.

The bivalent zinc can be incorporated into the infusion medium or cell culture medium using any method, for example, the above-described bivalent zinc compounds can be added to the infusion medium or cell culture medium in solid form and dissolved therein, although it is preferable to add such in the form of a solution such as in an aqueous solution, in an aqueous acidic solution or in an aqueous buffered solution. Similarly, it is preferable in the processes of the present invention to employ an aqueous solution of the bivalent zinc compounds.

The following example of the present invention is provided for illustrative purposes only and are in no way intended to limit the scope of the present invention. Unless otherwise indicated herein, all parts, percents, ratios and the like are by weight.

In the example, malignant transformed human cell lines obtained from the American Type Culture Collection, i.e., a human epithelial cervix carcinoma cell line, HeLa (ATCC No. CCL-2), a human adenocarcinoma carcinoma cell line of the colon, HT-29 (ATCC No. HTB-38) and a human adenocarcinoma carcinoma cell line of the breast, SK-BR-3 (ATCC No. HTB-30) were used. Non-transformed diploid human fibroblast cells (MRC-5, ATCC No. CCL-171) were employed as a control. The cell lines were obtained from the American Type Culture Collection in a frozen condition. In order to reactivate the cells, the cells were subcultured in McCoy 5A (mod.) medium (Serva Company) containing 15% (v/v) fetal calf serum.

The reactivated cells were allowed to reach the logarithmic growth phase and then detrypsinized and harvested. The harvested cells were washed twice with McCoy 5A (mod.) medium containing 15% (v/v) fetal calf serum and then resuspended in the same medium at a concentration of $10^6$ cells per ml. The correct cell concentration was determined using a Neubeuer-Turk count chamber in which the viability of the cells was also determined by means of Trypan blue coloring (see: Freshney, I.R., Culture of Animal Cells, Alan Riss Publ. Co., New York, N.Y. (1983)).

To tubes containing 200,000 HTB-30 cells or 200,000 HTB-38 cells or 200,000 CCL-2 cells was added Cell Culture Medium Nos. 1, 2, 3, 4, 5, 6, 7, 8 or 9 described below and the cells were incubated for 80 hours. In addition, to tubes containing 200,000 human fibroblast cells (MRC-5) were added Cell Culture Medium Nos. 1, 2, 3, 4, 5, 6, 7, 8 or 9 described below and the cells were incubated for 80 hours. All of the cells were incubated at 36.5°C (± 0.5°C), 5% $CO_2$ and 95% air. Five duplicate tubes were employed for each cell culture medium for each cell.

Cell Culture Medium No. 1 comprised McCoy 5A (mod.) medium plus 15% (v/v) fetal calf serum without the addition of bivalent zinc. McCoy 5A (mod.) medium only contains trace amounts of bivalent zinc, i.e., 0.0 ± 0.001 mg/l of $Zn^{++}$.

Cell Culture Medium No. 2 was identical to Cell Culture Medium No. 1 except that $ZnCl_2$ was added to a final concentration of 0.2 mg/l of $Zn^{++}$.

Cell Culture Medium No. 3 was identical to Cell Culture Medium No. 1 except that $ZnCl_2$ was added to a final concentration of 0.8 mg/l of $Zn^{++}$.

Cell Culture Medium No. 4 was identical to Cell Culture Medium No. 1 except that $ZnCl_2$ was added to a final concentration of 1.2 mg/l of $Zn^{++}$.

Cell Culture Medium No. 5 was identical to Cell Culture Medium No. 1 except that $ZnCl_2$ was added to a final concentration of 1.6 mg/l of $Zn^{++}$.

Cell Culture Medium No. 6 was identical to Cell Culture Medium No. 1 except that $ZnCl_2$ was added to a final concentration of 2.1 mg/l of $Zn^{++}$.

Cell Culture Medium No. 7 was identical to Cell Culture Medium No. 1 except that $ZnCl_2$ was added to

a final concentration of 2.8 mg/l of $Zn^{++}$.

Cell Culture Medium No. 8 was identical to Cell Culture Medium No. 1 except that $ZnCl_2$ was added to a final concentration of 3.5 mg/l of $Zn^{++}$.

Cell Culture Medium No. 9 was identical to Cell Culture Medium No. 1 except that $ZnCl_2$ was added to a final concentration of 4.0 mg/l of $Zn^{++}$.

After the end of the incubation time, the culture medium was removed and the cells were washed three times with Dulbecco buffer solution (see: Dulbecco, R.U., Vogt, M., J. Exp. Med. 98:167-182 (1954)). Next, the cells were dried and octyl phenol polyethylene glycol ether was added at a concentration of 0.2% (v/v) to each tube to which 20 $\mu$l of 1.0 N NaOH had been additionally added per ml.

After thorough mixing and allowing the samples to stand for 30 minutes at 20-25°C, 200 $\mu$l each of the resulting cell extracts were used for protein determinations according to the Lowry method (see: Lowry, O.H. et al., J. Biol. Chem. 193:265 (1951)).

The results obtained are shown in the Table below. The Control in the Table below refers to Cell Culture Medium No. 1 at 4°C to which no cells had been added. In the Table, the growth percentage is determined based upon Cell Culture Medium No. 1, which does not contain bivalent zinc above trace amounts, giving rise to a 100% growth value. All growth values are mean values from the five duplicate tubes ± the standard deviation.

The growth value as a percent of the Control was determined according to the following formula:

$$\% \text{ growth} = 100 \times \frac{\text{Absorption of Sample} - \text{Absorption of Control (initial value)}}{\text{Absorption of Sample in Medium No. 1} - \text{Absorption of Control (initial value)}}$$

In addition, in the Table, the mean adsorption values are determinative of the concentration of protein in the cells and were evaluated at 660 nm.

The mean absorption values for two concentrations of bovine serum albumin (BSA) were measured as shown below and used a control for the protein concentration determinations:

(a) BSA 100 $\mu$g/tube: 0.270 0.280 0.248 0.265

(b) BSA 50 $\mu$g/tube: 0.135 0.146 0.143 0.125

## TABLE

| Samples | HTB-38 | | HTB-30 | | CCL-2 | | Human Fibroblasts | |
|---|---|---|---|---|---|---|---|---|
| | Mean adsorption values | % growth | Mean adsorption values | % growth | Mean adsorption values | % growth | Mean adsorption Values | % growth |
| Control | 0.076 ± 0.004 | --- | 0.022 ± 0.007 | --- | 0.072 ± 0.006 | --- | 0.036 ± 0.006 | --- |
| Medium No. 1 | 0.580 ± 0.018 | 100 | 0.211 ± 0.027 | 100 | 0.736 ± 0.016 | 100 | 0.319 ± 0.025 | 100 |
| Medium No. 2 | 0.552 ± 0.021 | 94.4 ± 3.8 | 0.204 ± 0.023 | 96.3 ± 11.2 | 0.793 ± 0.035 | 108.6 ± 4.4 | 0.327 ± 0.034 | 102.8 ± 10.3 |
| Medium No. 3 | 0.596 ± 0.044 | 103.2 ± 7.4 | 0.269 ± 0.026 | 130.7 ± 9.6 | 0.929 ± 0.075 | 129.1 ± 8.1 | 0.299 ± 0.028 | 105.6 ± 9.7 |
| Medium No. 4 | 0.945 ± 0.071 | 172.5 ± 7.5 | 0.393 ± 0.020 | 196.3 ± 5.0 | 1.101 ± 0.040 | 155.0 ± 3.6 | 0.309 ± 0.018 | 104.2 ± 5.8 |
| Medium No. 5 | 0.807 ± 0.066 | 145.0 ± 8.2 | 0.268 ± 0.011 | 130.2 ± 4.1 | 0.921 ± 0.063 | 128.0 ± 6.8 | 0.327 ± 0.053 | 115.5 ± 10.2 |
| Medium No. 6 | 0.455 ± 0.037 | 75.2 ± 8.2 | 0.188 ± 0.016 | 87.8 ± 8.5 | 0.649 ± 0.060 | 86.9 ± 9.2 | 0.323 ± 0.010 | 114.1 ± 3.1 |
| Medium No. 7 | 0.425 ± 0.041 | 69.0 ± 9.6 | 0.164 ± 0.012 | 75.2 ± 7.3 | 0.588 ± 0.036 | 77.7 ± 6.1 | 0.326 ± 0.023 | 115.1 ± 7.1 |
| Medium No. 8 | 0.369 ± 0.028 | 58.2 ± 7.6 | 0.145 ± 0.004 | 65.1 ± 6.2 | 0.343 ± 0.017 | 40.8 ± 5.0 | 0.292 ± 0.015 | 103.1 ± 5.1 |
| Medium No. 9 | 0.364 ± 0.031 | 57.2 ± 8.5 | 0.142 ± 0.010 | 63.5 ± 7.0 | 0.371 ± 0.014 | 45.1 ± 3.8 | 0.291 ± 0.020 | 102.8 ± 6.9 |

0 291 164

The growth values shown in the Table above for HTB-38, HTB-30, CCL-2 and fibroblast cells are graphically illustrated in the Figure. The Figure shows that the growth rate, expressed as protein concentration, is inhibited for the transformed HTB-38 cells, HTB-30 cells and CCL-2 cells when grown in a basic cell culture medium containing from about 1.6 mg/l to about 4.0 mg/l of $Zn^{++}$ but is not inhibited for the non-transformed human fibroblasts.

Specifically, the Table and the Figure clearly demonstrate that cell line HTB-38 exhibits a 24.8% decrease in protein yield, when compared to Cell Culture Medium No. 1, when bivalent zinc is employed in the basic culture medium in an amount of about 2.1 mg/l of $Zn^{++}$. In addition, the results in the Table and Figure demonstrate that a maximum growth suppression is achieved when bivalent zinc is employed in an amount 3.5 mg/l of $Zn^{++}$ of the basic cell culture medium, i.e., cell line HTB-38 had a 41.8% decrease in protein yield when compared to Cell Culture Medium No. 1. A similar decrease is found for the HTB-30 and CCL-2 transformed cell lines but, there is no decrease for the control non-transformed fibroblasts.

While the present invention has been described in detail and with respect to specific embodiments thereof, it would apparent that changes and modifications can be made therein without departing from the spirit and scope thereof.

## Claims

1. A medium comprising a basic infusion medium or a basic cell culture medium, and additionally, bivalent zinc in an amount sufficiently high to suppress transformed cell growth.

2. A medium as claimed in claim 1, wherein the bivalent zinc is present in an amount of about 1 6 mg/l of $Zn^{++}$ or greater.

3. A medium as claimed in claim 2, wherein the bivalent zinc is present in an amount of from about 2.1 to 4.0 mg/l of $Zn^{++}$.

4. A medium as claimed in any one of claims 1 to 3 wherein the source of the bivalent zinc is a compound selected from the group consisting of non-toxic organic bivalent zinc compounds and non-toxic inorganic bivalent zinc compounds.

5. A medium as claimed in claim 4, wherein the non-toxic organic bivalent zinc compounds are zinc salts of organic compounds.

6. A medium as claimed in claim 6, wherein the non-toxic inorganic bivalent zinc compounds are selected from the group consisting of $ZnCl_2$, $ZnO$, $ZnCO_3$ and $ZnSO_4$.

7. An infusion medium as claimed in any one of the preceding claims wherein the basic infusion medium is selected from the group consisting of a physiological saline solution, a glucose solution, a fructose solution, whole blood fractions, plasma and plasma expanders.

8. A process for suppressing the growth of transformed cells in vitro comprising culturing transformed cells in the presence of a cell culture medium comprising a basic cell culture medium, and additionally bivalent zinc, in an amount sufficiently high to suppress cell growth.

9. The process for suppressing cells as claimed in claim 8, wherein the bivalent zinc is present in an amount of about 1.6 mg/l of $Zn^{++}$ or greater, and wherein the source of the bivalent zinc is a compound selected from the group consisting of $ZnCl_2$, $ZnO$, $ZnCO_3$ and $ZnSO_4$.

10. The use of bivalent zinc for the manufacture of a medicament for the suppression of tumor growth.

11. The use of bivalent zinc as claimed in claim 10 wherein the medicament additionally comprises one or more cytostatica.

0 291 164